# EUROPEAN PATENT SPECIFICATION

(11) **EP 2 503 957 B1**
(45) Date of publication and mention of the grant of the patent: **15.03.2017**
(21) Application number: 10795778.9
(22) Date of filing: 24.11.2010
(51) Int. Cl.: A61F 2/00

(54) **APPARATUSES FOR THE TREATMENT OF URINARY STRESS AND URGE INCONTINENCE**
VORRICHTUNG ZUR BEHANDLUNG VON BELASTUNGSHARNINKONTINENZ UND DRANGHARNINKONTINENZ
DISPOSITIFS POUR LE TRAITEMENT DE L'INCONTINENCE URINAIRE D'EFFORT ET D'URGENCE

(30) Priority: 24.11.2009 US 263854 P
(43) Date of publication of application: 03.10.2012
(73) Proprietor: Kimberly-Clark Worldwide, Inc., Neenah, Wisconsin 54956 (US)
(72) Inventor: ZIV, Elan, 52648 Ramat-Gan (IL); PERLE, Amir, 34677 Haifa (IL)
(74) Representative: Mabey, Katherine Frances
(86) International application number: PCT/IL2010/000985
(87) International publication number: WO 2011/064774

(56) References cited:
- WO-A2-2005/087154
- WO-A2-2008/010214
- WO-A2-2009/044394

## Description

### FIELD AND BACKGROUND OF THE INVENTION

The present invention relates generally to treating feminine medical conditions, for example by providing devices for the prevention of female incontinence.

Related teachings include: PCT/IL2004/000433 filed on May 20, 2004; PCT/IL2005/000304 filed on March 17, 2005; PCT/IL2005/000303 filed March 17, 2005; PCT/IL2006/000346 filed on March 16, 2006; PCT/IL2007/000893 filed on July 16, 2007; PCT/IL2008/001292 filed on September 24, 2008; U.S. Provisional Patent Application No. 60/719,422 filed on September 22, 2005; U.S. Provisional Patent Application No. 60/762,059 filed on January 25, 2006; and, U.S. Provisional Patent Application No. 60/960,492 filed on October 1, 2007.

Urinary incontinence is a widespread problem among females. It is estimated that up to 50% of women occasionally leak urine involuntarily, and that approximately 25% of women will seek medical advice at some point in order to deal with the problem. Stress incontinence, the most common type of urinary incontinence, refers to the involuntary loss of urine resulting from an abdominal pressure rise. When involuntary urination occurs, it often happens because of a rise in pressure in the bladder for which there is no compensating counter-pressure from the bladder neck or urethra. This is usually the result of the abnormal descent of the bladder neck and the urethra into a low position, away from the intra-abdominal pressure system. Known as "hypermobility", this is the result of some injury to the support mechanism which normally keeps the urethra and the bladder neck in a raised position, along the backside of the pubic bone.

The lowering of the bladder neck and the urethra that occur, for example, when a woman coughs, sneezes, or laughs, causing involuntary leakage of urine. While many different factors may contribute to the development of stress incontinence, it is most
prevalent among women ages 35-65 and those who have had multiple vaginal deliveries.

Stress incontinence is both aggravating and unpleasant for women, and it can also be embarrassing. Many women wear sanitary pads or diapers in order to deal with incontinence, though this is not a real solution to the problem and it can be very inconvenient and unreliable. Surgical treatment may involve, among others, elevation of the anterior vaginal wall (Anterior Colporrhaphy), securing the paraurethal tissues to the periosteum of the pubic bone (Marshall-Marchetti-Krantz operation), or elevation of the paracervical vaginal anterior wall to the Coopers ligament (Burch Colpo suspension) in order to elevate the bladder neck above the level of the pelvic floor and thereby distribute pressure equally to the bladder, the bladder neck, and the mid-urethra. Recently, a procedure known as "TVT" ("Tension Free Vaginal Tape") was developed, in which a mesh tape is implanted underneath the urethra (usually mid-urethra), creating a hammock on which the urethra may kink during a rise in intra-abdominal pressure. However, surgery is only suitable for severe cases, and the majority of women experiencing incontinence does not need, and certainly would rather avoid, surgical solutions.

One modality of non-surgical treatment involves the use of devices that are inserted into the vagina, either by a medical practitioner or by the woman herself. Most devices are designed to apply pressure against the bladder neck so as to inhibit or completely block the flow of urine through the urethra. A variety of such devices are known in the art. For example, refer to U.S. Publication No. 2002/0183711 to Moser, entitled, "Urinary Incontinence Device"; U.S. Patent No. 6,739,340 to Jensen, et al., entitled, "Device for prevention of involuntary urination"; U.S. Patent No. 6,679,831 to Zunker, et al., entitled, "Resilient incontinence insert and a method of making the same"; U.S. Patent No. 6,460,542 to James, entitled, "Female incontinence control device"; U.S. Patent No. 6,413,206 to Biswas, entitled, "Intra-vaginal device"; U.S. Patent No. 5,785,640 to Kresch, entitled "Method for Treating Female Incontinence"; U.S. Patent No. 5,771,899 to Martelly, et al., entitled, "Pessary"; U.S. Patent No. 5,618,256 to Reimer, entitled, "Device for Arrangement in the Vagina for Prevention of Involuntary Urination with Females and an Applicator for use in Insertion of the Device"; U.S. Patent 5,417,226 to Juma, entitled, "Female Anti-Incontinence Device"; U.S. Patent No. 5,386,836 to Biswas, entitled, "Urinary Incontinence Device"; U.S. Patent No. 5,007,894 to Enhorning, entitled, "Female Incontinence Device"; and U.S. Patent No. 4,920,986 to Biswas, entitled, "Urinary Incontinence Device".

One problem with many of the above listed devices is that they completely block the urethra and thus they need to be removed or collapsed in order to allow the woman to urinate. To overcome this drawback, vaginal devices have been developed having specialized shapes that do not completely block the urethra but these devices tend to be large, uncomfortable, and intrusive. They also tend to cause irritation or soreness to the vagina.

Another common shortcoming is that most devices known in the art also tend to be difficult or painful or uncomfortable to insert and/or remove. In order to correctly inhibit urine flow, the device needs to be properly positioned in the vaginal canal. As a result, a doctor may be required to properly position the device. In most cases, the device is adapted for remaining in the vagina for a prolonged period of time (due to the time and expense of requiring a trained medical professional to insert the device). However, when positioned in the vagina for an extended period of the time, the device may cause vaginal infections, pressure ulcers, and/or bleeding.

### SUMMARY OF THE INVENTION

The present invention provides an apparatus for insertion into a human vagina for treating urinary incontinence as claimed in claim 1.

Where in the following the word invention is used and/
or features are presented as optional, this should be interpreted such
a way that protection is sought for the invention as claimed.

An aspect of some embodiments of the invention relates to providing an intra-vaginal device which is comfortable to wear in an at-rest state but which provides incontinence prevention during a high stress event. In an embodiment of the invention, the device is constructed from a resilient material and in a form which, when placed under stress during a high stress event, causes a counter-force to be applied effective to prevent incontinence. In an embodiment of the invention, the device is adapted to apply a desired force by endowing at least a portion of the device with a predetermined effective elasticity. In an embodiment of the invention, the device is adapted to apply a desired force by shaping and/or sizing at least support arms of the device exhibit predetermined performance characteristics.

An aspect of some embodiments of the invention relates to providing an intra-vaginal device for treating urinary incontinence adapted for reduced profile storage, assembly and/or comfort with beveled features. The supporting arms of the device are beveled. The anchoring arms of the device are also beveled.

In some embodiments of the invention, the beveled arms permit the device to be inserted into and/or stored in an applicator of smaller size than would be used if the arms were not beveled.

The beveled arms form a tunnel, which may be a quadrilateral tunnel, when compressed towards a central axis of the device which allows for a rod to be inserted through the tunnel and into a channel in the device itself for urging the device into the applicator during assembly.

In some embodiments of the invention, the beveled arms enhance the comfort of the user of the device by spreading out the forces exerted on the device by the vaginal wall and/or by reducing the likelihood of a single corner of the device being the focus of the forces exerted on the device by the vaginal wall.

In an embodiment of the invention, at least one of the supporting and/or anchoring arms is provided with a tip adapted to prevent rotational motion of device in the vagina once in the proper deployed, support-rendering position, for example by being sized and/or shaped to induce the vaginal wall to at least partially envelop the tip, thereby preventing rotational movement. Optionally, the tip is also adapted to enhance comfort to the user, for example by being beveled and/or rounded.

The device features at least four aspects of flexibility, in some embodiments of the invention. First, a central portion of the device, the node or neck, is flexible allowing the device to bend to conform to the longitudinal shape or geometry of the user's vagina. Second, each individual arm is capable of flexing so that its respective tip can be positioned in accordance with the vaginal topography where that tip is located and/or without regard and/or separate from the positioning of tips of other arms of the device. This flexing includes towards or away from the central longitudinal axis of the device and also at any other angle to the longitudinal axis of the device. Third, the flexibility of the neck in combination with the flexibility of the individual arms allows for flexing of the device in axes perpendicular to the longitudinal axis of the vagina (i.e. horizontal rotation). Fourth, each arm is adapted to twist clockwise and/or counterclockwise around its own longitudinal axis.

In an embodiment of the invention, the device can be inserted in any orientation and still render effective sub-urethral support when deployed. This is at least partly due to at least one of the four aspects of flexibility described above and/or to the symmetry of the device design with respect to the central axis of the device. For example the flexibility of the device causes it to migrate in the vagina upon initial deployment until it settles into proper support-rendering position (wherein two support arms are positioned on either side of the urethra, as described below), in an embodiment of the invention.

In some exemplary embodiments of the invention, the device counters force applied to it by increasing counter-force as force is applied.

An applicator is used for storing and/or inserting and/or deploying the device, in an exemplary embodiment of the invention. In an embodiment of the invention, the device begins rendering support immediately upon deployment from the applicator.

An aspect of some embodiments of the invention relates to a method for packaging a urinary incontinence device kit and/or product. The rod is inserted into a channel and is used to urge the device into an applicator during packing of the commercial product. Supporting arms, which are nominally in a radially expanded configuration, compress as they come into contact with the housing or a ring shaped guide of the applicator and as the device is pushed into the space within the applicator housing, in an embodiment of the invention. Beveled/squared-off edges of the supporting arms create a quadrilateral tunnel for the rod which is being used to perform the pushing when the supporting arms are compressed to fit into the applicator. Once the device has been fully enclosed by the applicator housing, the rod is retracted from the device.

There is thus provided in accordance with an exemplary embodiment of the invention, an apparatus for insertion into a human vagina for treating urinary incontinence, comprising: a supporting section which renders sub-urethral support by actively providing a counter-force to at least a vaginal wall in response to a high stress event.

In an exemplary embodiment of the invention, the supporting section has a maximal diameter at which the apparatus renders a minimal applied force.

In an exemplary embodiment of the invention, the maximal diameter is between 32 mm and 48.5 mm.

In an exemplary embodiment of the invention, the supporting section has a minimal diameter at which the apparatus renders a maximal applied force.

In an exemplary embodiment of the invention, the minimal diameter is between 16 mm and 41.5 mm.

In an exemplary embodiment of the invention, the diameter of the supporting section is reduced up to 5.3 mm in response to a high stress event of 10 g. Optionally, the diameter of the supporting section is reduced up to 10.6 mm in response to a high stress event of 20 g. Optionally, the diameter of the supporting section is reduced up to 20 mm in response to a high stress event of at least 45 g.

In an exemplary embodiment of the invention, the apparatus is adapted for use by a variety of patients by having a low slope, wherein the slope is the force applied by the apparatus divided by the amount of medial deflection.

In an exemplary embodiment of the invention, the low slope means 2.5 or below. Optionally, low slope means 1.9 or below.

In an exemplary embodiment of the invention, the supporting section renders between 10 g and 50 g of sub-urethral support at rest.

In an exemplary embodiment of the invention, the apparatus is offered in a plurality of sizes depending on the individual needs of the patient.

In an exemplary embodiment of the invention, the plurality of sizes range from 36 mm in diameter to 50 mm in diameter.

In an exemplary embodiment of the invention, the apparatus is offered in a plurality of hardnesses. Optionally, the hardnesses range from Shore A 40 to Shore A 70.

In an exemplary embodiment of the invention, the apparatus is offered with a plurality of slopes, wherein the slope is the force applied by the apparatus divided by the amount of medial deflection. Optionally, the slopes range from 1.9 to 7.7.

There is further provided in accordance with an exemplary embodiment of the invention, an apparatus for insertion into a human vagina for treating urinary incontinence, comprising: an intermediate section; an anchoring section located on one side of the intermediate section provided with a plurality of beveled anchoring arms adapted to prevent movement of the apparatus into the vagina; and, a supporting section located on the opposite side of the intermediate section from the anchoring section provided with a plurality of beveled supporting arms adapted to prevent movement of the apparatus out of the vagina and to provide sub-urethral support.

The apparatus further comprises a channel positioned along the central axis of the device and adapted for insertion of a rod therethrough.

In an exemplary embodiment of the invention, at least one of the anchoring arms and the supporting arms are provided with tips adapted to induce the vaginal wall to envelop the tips to prevent rotational motion of the apparatus in the vagina.

In an exemplary embodiment of the invention, at least one of the anchoring arms and the supporting arms are flexible.

In an exemplary embodiment of the invention, the intermediate section is adapted to conform to the longitudinal geometry of the vagina by being flexible around the natural arch of the vagina.

In an exemplary embodiment of the invention, a square tunnel adapted for insertion of a rod therethrough is formed by the bevels of the plurality of supporting arms when the supporting arms are maximally compressed towards a central axis of the apparatus.

In an exemplary embodiment of the invention, the apparatus further comprises a cover. Optionally, the cover encloses the intermediate, anchoring and supporting sections.

In an exemplary embodiment of the invention, the apparatus further comprises a removal string attached to the cover. Optionally, force applied to the removal string exerts a compressive force on at least the supporting section by tightening the cover at least around the supporting section.

In an exemplary embodiment of the invention, the intermediate section flexes to adapt the apparatus to the shape of the vagina.

In an exemplary embodiment of the invention, each of the plurality of arms of the supporting section supplies counter-force to the vaginal wall in proportion to the force applied to the arm by the vaginal wall.

In an exemplary embodiment of the invention, each of the plurality of supporting arms flexes independently of the other supporting arms.

In an exemplary embodiment of the invention, each of the plurality of anchoring arms flexes in response to force from the vaginal wall.

In an exemplary embodiment of the invention, each of the plurality of anchoring arms flexes independently of the other anchoring arms.

In an exemplary embodiment of the invention, the apparatus further comprises an applicator adapted to store and deploy the apparatus into the vagina.

In an exemplary embodiment of the invention, the beveling of the plurality of anchoring and supporting arms is adapted to match the circumference of an applicator housing of the applicator.

In an exemplary embodiment of the invention, the plurality of beveled supporting arms is arranged symmetrically around a central axis of the device.

In an exemplary embodiment of the invention, the device can be inserted into the vagina in any radial orientation around the central axis to provide the sub-urethral support due to its symmetry.

In an exemplary embodiment of the invention, each of the plurality of beveled supporting arms is sufficiently flexible to twist at least one of clockwise and counter-clockwise around its own longitudinal axis.

In an exemplary embodiment of the invention, each of the plurality of beveled supporting arms is at least flexible towards a central axis of the device.

In an exemplary embodiment of the invention, each of the plurality of beveled supporting arms is provided with a tip.

In an exemplary embodiment of the invention, the tip is at least one of beveled and rounded.

There is thus provided in accordance with an exemplary embodiment of the invention, an apparatus for treating urinary incontinence, comprising: a supporting section with a slope of 2.5 or lower, wherein the slope is the force applied by the apparatus divided by the amount of medial deflection. Optionally, the slope is 1.9 or lower.

There is thus provided in accordance with an exemplary embodiment of the invention, an apparatus for treating urinary incontinence, comprising: a supporting section which renders between 10 grams and 50 grams of sub-urethral support at rest. In an exemplary embodiment of the invention, the supporting section renders between 60 grams and 230 grams of sub-urethral support during a high stress event.

There is thus provided in accordance with an exemplary embodiment of the invention, a method of packaging a urinary incontinence device, comprising: inserting a rod into a channel in the device located along a central axis of the device, wherein the channel is accessed by the rod through a tunnel created by beveled supporting arms of the device; urging the device towards an applicator housing using the rod; compressing the supporting arms of the device towards the central axis while pushing the device into the applicator housing; and, retracting the rod when the device is fully enclosed by the applicator housing.

In an exemplary embodiment of the invention, the method further comprises wrapping the applicator in a product wrapper after the device has been inserted therein.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:
FIGs. 1A-1C are perspective views of a device for treating urinary incontinence, in accordance with an exemplary embodiment of the invention;
FIGs. 1D-1E are cross-sectional views showing the beveled nature of the supporting arms, in accordance with an exemplary embodiment of the invention;
FIG. 2 is a perspective view of a device inside a cover provided with a removal string, in accordance with an exemplary embodiment of the invention;
FIGs. 3A-3C are perspective views of an applicator for a device for treating urinary incontinence, in accordance with an exemplary embodiment of the invention;
FIG. 4 is a side view of the component parts of a product package for a device for treating urinary incontinence, in accordance with an exemplary embodiment of the invention;
FIG. 5 is a plan view showing a possible relationship of the arms to the central axis of the device when the device is *in situ,* in accordance with an exemplary embodiment of the invention;
FIG. 6 is a flowchart illustrating a method for manufacturing, assembly and packaging of an incontinence device, in accordance with an exemplary embodiment of the invention;
FIG. 7 is a chart illustrating exemplary device specifications, in accordance with an exemplary embodiment of the invention;
FIG. 8 is a device performance graph correlating force exerted to device size and diameter and hardness, in accordance with an exemplary embodiment of the invention;
FIG. 9 is a graph correlating device diameter to force exerted during selected high stress moments, in accordance with an exemplary embodiment of the invention;
FIG. 10 is a table showing exemplary performance ranges for medial deflection and horizontal rotation (left/right from central axis), in accordance with an exemplary embodiment of the invention; and,
FIG. 11 is a table showing performance characteristics for exemplary basic device configurations, in accordance with an exemplary embodiment of the invention.

### DESCRIPTION OF EMBODIMENTS OF THE INVENTION

The present invention, in some embodiments thereof, provides a device, and variations of the device, for the treatment of urinary incontinence in females. In embodiments of the invention, the devices described herein are adapted to provide tension-free, incontinence treating support perpendicularly to the urethra (*i.e.* across the urethra).

In an embodiment of the invention, the device is adapted to be stable in the vagina, without significant longitudinal and/or rotational movement within the vagina. For example, supporting and anchoring arms of the device, described in more detail below, are designed to resist longitudinal movement in the vagina. As another example, the tips of the arms are designed to resist rotational motion by working with the natural behavior of the vaginal wall, for example by being sized and/or shaped to induce the vaginal wall to at least partially envelop the tip, thereby preventing rotational movement. Stability is also enhanced by using arms to provide contact between the device and multiple points located spatially around the device on the vaginal wall. In an embodiment of the invention, proper support-rendering position of the device is considered to be where two supporting arms position themselves one on each side of the urethra while at least one other arm provides opposing force to the device when the two supporting arms are subjected to force from the urethra during high-stress events that cause the urethra to drop in the vagina.

In some embodiments of the invention, the device is adapted to be disposable, worn only for a relatively short period of time and then discarded and replaced with a new device (if needed). Alternatively, the device is recycled for use by sterilizing it in between uses. The device of the present invention is simple and easy to use, and is optionally inserted effortlessly in the same user-friendly and familiar manner that a tampon is inserted into the vagina during menstruation, for example using an applicator. In an embodiment of the invention, the device can be inserted in any orientation since the device will naturally migrate into a correct treatment position as a result of the device geometry. As opposed to large and intrusive devices of the prior art, the device of the present invention is small (exemplary sizes indicated below), comfortable, and, once inserted, the woman need not think about it again until it is removed. As with insertion, removal is accomplished in a similar manner as a tampon, by pulling on a string, in an embodiment of the invention.

In some embodiments of the invention, treatment of incontinence is effectuated by deploying the device, which utilizes at least one of Sub Urethral Tension Free Support (SUTFS), colpo-elevation and/or colpo-distension mechanisms, described in more detail in related application WO2008010214. In some embodiments of the invention, more than one of these principles of operation are used in combination by a device in order to treat incontinence.

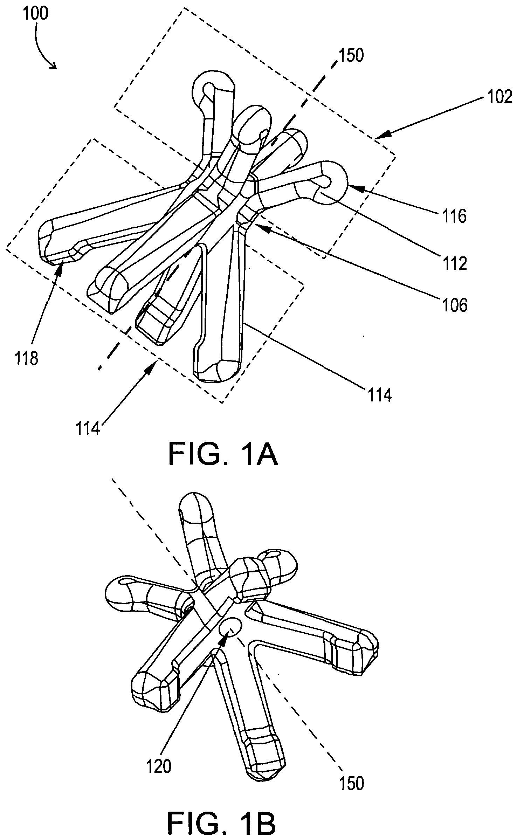

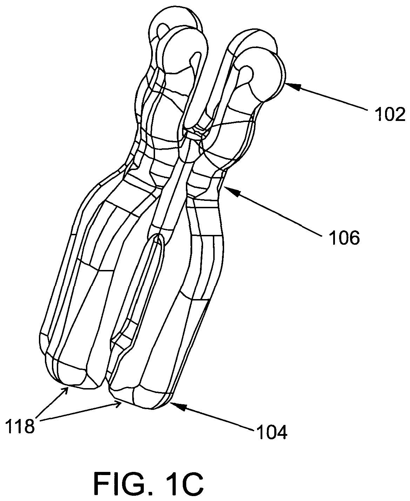

Referring to Fig. 1A, a perspective view of an exemplary embodiment of the incontinence device 100 is shown. For ease of description, the device 100 is arranged around a central axis 150 and divided into three basic elements. A top section 102, inside the dashed box, is provided which serves as the "anchoring" element, for stabilizing the device 100 within the vagina. There are at least three types of anchoring, axial anchoring which acts in the direction along the central axis of the vagina, and radial anchoring which acts side-to-side or substantially perpendicular to the central axis of the vagina and/or rotational anchoring, described in more detail below. A bottom section 104 is provided which serves as the "supporting" element, for generating support. In some embodiments of the invention, the roles of the anchoring and supporting elements could be switched or shared. In some embodiments of the invention, support is generated at a sub-urethral location, for example mid-urethra. Alternatively, additionally and/or optionally, support is generated at the bladder neck. In some embodiments of the invention, the bottom supporting section 104 provides at least one form of anchoring described above to help anchor device 100 in position.

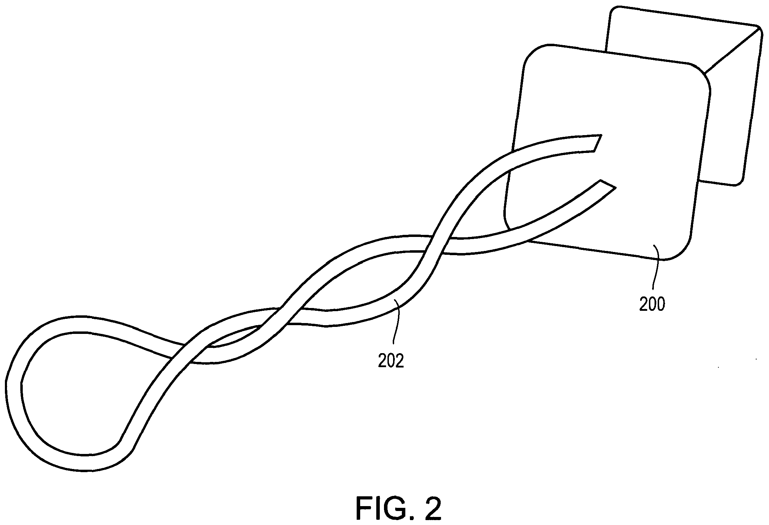

Also, an intermediate section 106 is optionally provided which acts as a "node" and which connects anchoring 102 and supporting 104 sections. The node 106 of device 100 has a length which is only a small portion of the overall length of the device, in some embodiments of the invention. In some embodiments of the invention, the length of the node is less than 15% of the entire length of the device, not including the removal string, described in more detail with respect to FIG. 2. In some embodiments of the invention, the length of the node is less than 20% of the entire length of the device. In other embodiments of the invention, the length of the node is less than 30% the entire length of the device. In some embodiments of the invention, a node which is short relative to the entire length of the device relative to a same length device with a long node, allows for more flexibility in varying the stiffness, the comfort, and the size of device 100.

In an exemplary embodiment, the anchoring element 102 and the supporting element 104 have four (4) arms 112 and 114, respectively. Optionally, these elements have more or less arms. In an exemplary embodiment of the invention, four arms are provided to each section in which two generally exert pressure towards the anterior vaginal wall, and two generally exert pressure towards the posterior vaginal wall adjacent the bowels. The distal part of the urethra extends into the vagina, forming a recess between the urethral bulge and the vaginal wall. The two support arms which exert pressure anteriorly fit within these natural recesses on either side of the urethra, in some embodiments of the invention. Optionally, the anchoring and supporting elements are provided with more or less arms. For example, the anchoring element could have more arms if there is concern about unwanted movement of device 100. In an exemplary embodiment of the invention, the anchoring element 102 does not apply significant pressure to the wearer's vagina and/or urethra, thereby enhancing comfort. The elements of the device 100 function as an internal support structure for a cover 200, depicted in FIG. 2, in some embodiments of the invention. It should also be noted that for certain women, the described devices herein can also be used as a treatment or part of a treatment for prolapse.

Anchoring arms 112 of the device prevent device 100 from moving unintentionally out of position. In some embodiments of the invention, the supporting arms 114 contribute to at least one of the three types of anchoring described above, for example rotational anchoring. In an exemplary embodiment of the invention, arms 112 are flexible and/or elastic and/or resilient. This flexibility enhances the anchoring arms' 112 ability to prevent motion of device 100 further into the vagina. As force strives to exert itself on device 100, and move it further into the vagina, the flexible anchoring arms 112 tend to spread apart. This spreading action of the anchoring arms 112 increases the friction between device 100 and the vaginal wall, preventing movement. While the arms 112 are flexible, it should be noted that they are rigid enough and/or are configured to spread to prevent unwanted motion of the device 100 towards the entrance of the vagina. Optionally, the arms 112 are rigid but the node is flexible, the node thus providing flexible anchoring and support.

Movement towards the vaginal opening is resisted by the support arms 114 which tend to widen radially when pulled outwardly. These features work with the tenting behavior of the vaginal walls described above, which also helps to maintain the device 100 in place, as described in more detail below.

In an embodiment of the invention, anchoring arms 112 are shorter than supporting arms 114, as shown in FIG. 1A. In an embodiment of the invention, anchoring arms 112 are a consistent size in a line of different products of device 100 (such as described below) even though the supporting arms 114 may vary in size and/or performance. Anchoring arms 112 are the same size, in some embodiments of the invention, to ease manufacturing considerations. Optionally, the anchoring arms 112 and supporting arms 114 are the same size. In some embodiments of the invention, the tips 116 of anchoring arms 112 are rounded or spherical in nature, to provide smooth surfaces (i.e. no corners or points) for the tenting of the vaginal wall.

An additional optional feature of the anchoring arms 112 of the device 100 is that they operate remotely, relative to the longitudinal axis of the vagina, from the support arms 114.

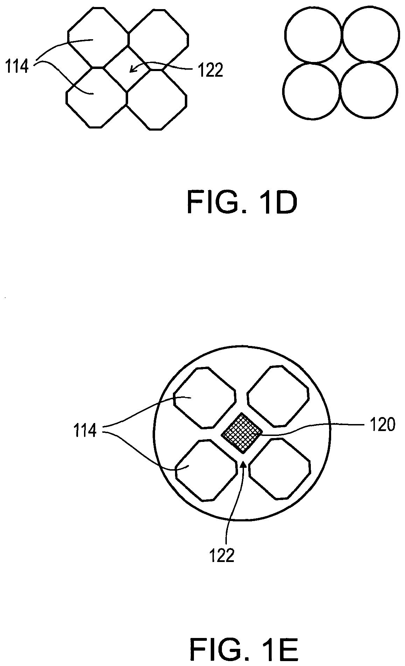

In an exemplary embodiment of the invention, the tips 118 of supporting arms 114 and/or corners of device 100 are blunted by a beveled edge both along the arms 112, 114 and at the tips 118, as shown more clearly in FIG. 1B. Optionally, tips 118 are slightly rounded and/or have a beveled edge. In an embodiment of the invention, the beveled edge of the supporting arms 114 reduces the overall circumference of the device, relative to a completely spherical cross section, when it is in a compressed mode for packaging within an applicator. The difference between a spherical cross-section and a device with a beveled cross section is shown in FIG. 1D. FIG. 1E shows a device 100 with a beveled cross-section in an applicator.

Also shown in FIG. 1B is a channel 120 which transects the device 100, whereby device 100 can be "held" during manufacturing and/or assembly by placing a rod through channel 120. Channel 120 is optionally circular in shape. Optionally, channel 120 is quadrilateral shaped. As shown in FIG. 1E, the channel 120 is accessible through the quadrilateral shaped tunnel 122 created by the beveled/squared-off edges of supporting arms 114 when compressed for packaging in the applicator. In an embodiment of the invention, channel 120 is no bigger in circumference and/or dimension than tunnel 122.

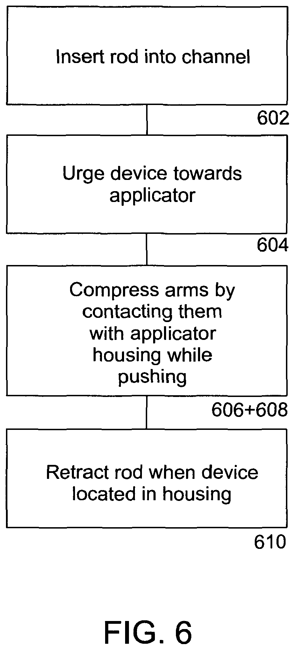

The rod which is inserted (602) into channel 120 is used to urge (604) device 100 into an applicator during packing and/or manufacturing of the commercial product, as shown in FIG. 6. Supporting arms 114, which are nominally in a radially expanded configuration, compress (606) as they come into contact with the housing of the applicator or external ring and as device 100 is pushed (608) into the space within the applicator housing. As described above and shown in FIG. 1E, the beveled/squared-off edges of supporting arms 114 create tunnel 122 for the rod which is being used to perform the pushing (608) when the supporting arms 114 are compressed (606) to fit into the applicator. Once the device has been fully enclosed by the applicator housing, the rod is retracted (610) from the device.

Devices of all sizes have the same length of arms (both anchor and support arms) in some embodiments of the invention, and/or they all have the same total length when completely squeezed inwardly, in an embodiment of the invention. In an exemplary embodiment of the invention, the difference between sizes is in the resting angle at which support arms protrude outwardly relative to the central axis 150 of the device 100. "Larger" size devices have a larger radial spread angle, hence they are "shorter" when put next to a smaller size device (i.e. a device which does not radially spread its supporting arms 114 as much). One potential advantage for such a design is to allow all devices no matter the radial spread angle to be inserted into a one size of applicator.

In an exemplary embodiment of the invention, anchoring arms 112 force device 100 to remain *in situ* within the vagina, unable to substantially move inwards or outwards, or to rotate. One reason this occurs, in some embodiments, is as a result of the special tendency of vaginal walls to collapse and form an occluded lumen. The arms of the device 100 cause "tenting" of the walls on top of them with resultant sagging of the walls around the device 100, thereby stabilizing the device 100. Supporting arms 114 cause elevation of the tissues around the urethra, optionally mid-urethra, or bladder neck acting as a hammock. This hammock supports the urethra in a tension free manner, much like the TVT operation. In a woman who leaks urine during a stressful event (when abdominal pressure rises during coughing, sneezing, etc.), the urethra sags down but meets the hammock in its mid part. The meeting of the urethra and the hammock causes an elevation (sort of like a kinking of the urethra) of the intra-urethral pressure with resultant urinary continence.

Various design aspects of device 100 encourage stability of the device 100 in the vagina, including: the longitudinal design of the device which incorporates anchoring arms 112 adapted to prevent movement of the device deeper into the vagina and/or supporting arms 114 adapted to prevent movement of the device towards the entrance to the vagina; specially adapted arm tips to resist rotational movement of the device as they dig into the vaginal wall; an overall design which takes advantage of the vaginal tenting phenomenon; and, a multi-dimensional aspect which allows various arms of the device to contact multiple and/or opposing vaginal surfaces concurrently.

In an embodiment of the invention, the flexibility of various components of device 100 is designed both for function and for comfort to the user. Device 100 is adapted with four aspects of flexibility which assist in accomplishing these goals of function and comfort, in an embodiment of the invention. For example, the node 106 of device 100 enables flexibility between its anchoring 102 and supporting 104 sections, enabling device 100 adjustment to the arch structure of the vagina, as well as adjustment to any position taken by the patient (*e.g*. standing, sitting, flexion etc.) during daily activity. By being able to flex at the node 106, pressure exerted on the vaginal wall is reduced in relation to a device that doesn't flex at the node.

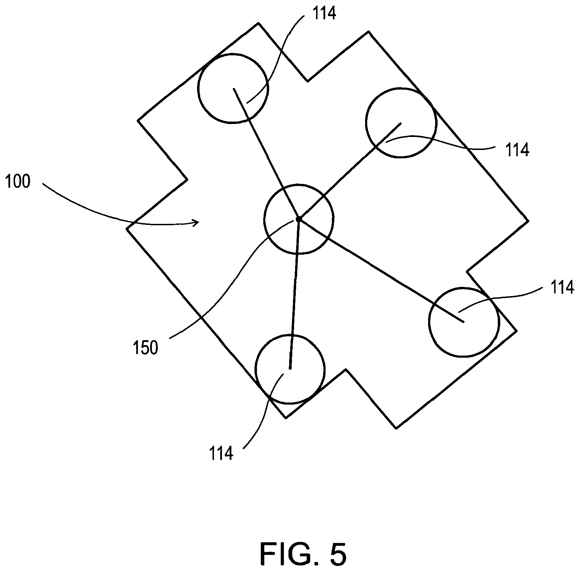

Another flexibility aspect of the device relates to providing efficacy and comfort across varying vaginal planes, for example where the arms are adapted to contact the vaginal wall at varying locations and/or angles of incidence, relative to each other, away from the central axis 150, as shown in FIG. 5, allowing device 100 to be adaptable to varying vaginal topography/geometries by rotating either left or right from the central axis of each arm.

A related aspect involves the ability of each of the arms to perform medial flexion, wherein each arm is flexible towards the central axis 150 when compressed by the vaginal wall enabling the adjustment of the device to various vaginal diameters.

A fourth flexibility aspect is the feature that each arm operates individually, for example each arm being able to twist clockwise and/or counterclockwise around its own axis and/or as described elsewhere herein, allowing device 100 to overcome vaginal structural variability from one point of contact to the next. It should be noted, that in some embodiment of the invention, at least one of these four featured aspects of flexibility allow device to render effective support regardless of vaginal dimension, vaginal shape, vaginal depth and/or through multiple planes.

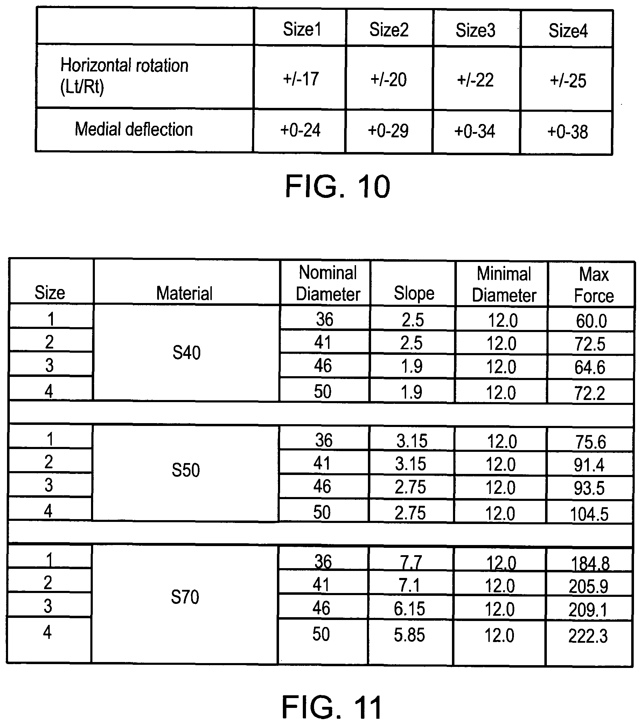

FIG. 10 is a table showing exemplary performance ranges for medial deflection (distance the tip of an arm or arms travel towards the central axis 150 of the device) and horizontal rotation (left/right movement perpendicular to central axis 150), in accordance with an exemplary embodiment of the invention. Numbers shown are in millimeters. It should be understood that horizontal rotation means rotation in axes perpendicular to the longitudinal axis of the vagina, particularly "right" and "left" when viewing the vagina in the longitudinal axis extending from the vaginal opening to the cervix. The ranges shown in FIG. 10 are amounts in mm that each arm could deviate from its natural position relative to the central axis of device 100. Regarding medial deflection, it should be noted that because when an arm is deflected medially, there is most often a corresponding arm on the other side of device which is also deflected medially (but maybe not the same amount), therefore in an embodiment of the invention, medial deflection numbers (in mm) are divided by two in order to approximate medial deflection for a single arm. It should also be noted that the numbers for medial deflection in table represent the full amount of deflection for a single arm assuming the corresponding arm does not move at all. In addition, in some embodiments of the invention, the maximum amount of medial deflection is dictated by two opposing arms contacting each other, preventing further medial deflection. In some embodiments of the invention, the minimum device diameter that can be achieved is 12 mm, with each arm representing a 6 mm portion of that total.

The device 100 is optionally made in a plurality of sizes and/or to exhibit specific performance characteristics, such as radial expansion of the supporting arms. This may be done to create a line of products offered commercially. In some embodiments of the invention, the diameter of the anchoring section ranges from 30mm to 33mm. In some embodiments of the invention, the diameter of the supporting section ranges from 34mm to 52mm. Different versions of device 100 are optionally made of different materials and/or materials exhibiting different performance characteristics, for example hardness. In some embodiments of the invention, device 100 is constructed of a material or materials which exhibit a Shore A hardness of 30-80. For example, device 100 can be manufactured in multiple hardnesses, including 40, 50 and 70.

In some exemplary embodiments of the invention, the device 100 is constructed from a single piece (Monoblock) and in other exemplary embodiments of the device the anchor section and support section are provided as separate pieces (bi-polar) which are attached to form the device. Optionally, each element, supporting or anchoring, is constructed of two or more pieces.

Referring to FIG. 2, a perspective view of device 100 completely inside a cover 200 provided with a removal string 202 is shown, in accordance with an exemplary embodiment of the invention. Cover 200 is optionally any of the covers described in PCT/IL2004/000433; PCT/IL2005/000304; PCT/IL2005/000303; PCT/IL2006/000346; PCT/IL2007/ 000893; PCT/IL2008/001292; U.S. Provisional Patent Application No. 60/719,422; U.S. Provisional Patent Application No. 60/762,059; and, U.S. Provisional Patent Application No. 60/960,492.

In some embodiments of the invention, cover 200 is smooth. In some embodiments of the invention, cover 200 is non-woven, for example constructed of 33gsm non-woven Fiberweb®, Catalog No. 097YLJO09P or any other non woven, for example made of 50% PP and 50 % PE. Optionally, cover 200 is comprised of nylon. Optionally, cover 200 and removal string 202 are constructed of the same unitary piece of material and/or at the same time and/or in the same process. Optionally, cover 200 is constructed of a non-absorbent material. In some embodiments of the invention, cover 200 is flexible and/or stretchable. In some embodiments of the invention, cover 200 is stitched and/or sutured together. Optionally, the stitches and/or sutures are inside cover 200, opposite the vaginal wall. In an embodiment of the invention, cover 200 and/or device 100 is adapted to allow the free flow of vaginal discharge.

In some embodiments of the invention, removal string 202 is comprised of cotton but it is optionally made of various materials, some of which allow for absorbing fluids and some of which do not. In some embodiments, removal string 202 is made of non-absorbent polyurethane, optionally with a coating of silicone film to provide a smooth surface to removal string 202. In some embodiments of the invention, removal string is silicone coated, braided polyester, an example of which is manufactured by Ashaway.

In some embodiments of the invention, removal string 202 is 14cm-16cm long, although the length can be varied in different device configurations. In an exemplary embodiment of the invention, removal string 202 is secured to cover 200 in a position whereby a pulling force towards the vaginal introitus is substantially evenly distributed over the cover 200 as it collapses the arms 114 of device 100 within the vagina, in an embodiment of the invention. Optionally, this position is in the center of the cover as shown in FIG. 2. Cover 200 is manufactured, in some embodiments of the invention, by cutting the appropriate shape and welding the cut cover material together, as shown and described with respect to FIGS. 22A-22C of PCT/IL2007/ 000893.

In some embodiments of the invention, cover 200 reduces friction between the device 100 and the vaginal wall. Pulling the removal string 202 causes tensioning of cover 200, in some embodiments of the invention. Tensioning of cover 200 results in the straightening of the vaginal walls. The straightening of the vaginal walls reduces the tent-like effect described above and relieves tension applied to the device 100, allowing for an easy and smooth removal of the device 100 from the vagina. Furthermore, pulling on removal string 202 causes the collapse of arms 114 at least slightly towards the central axis 150 as a result of the force applied to them by cover 200, thereby reducing the radial diameter of device 100 and allowing for an easy and smooth removal of the device 100 from the vagina.

Besides being used as an aid to removal of the device, an optional additional use of the cover is to act as a sling stretched between support arms 114, supplying sub-urethral tension free support to the urethra, as with the TVT operation.

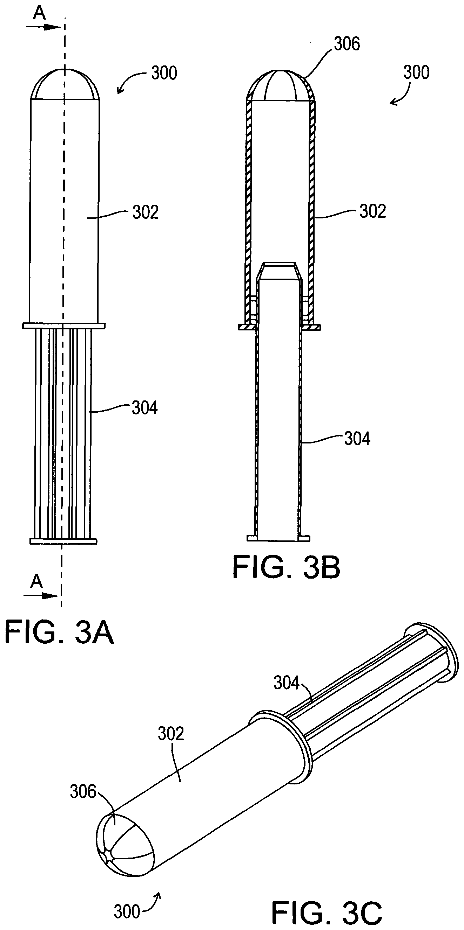

Referring to FIGS. 3A-3C, perspective views of an applicator 300 for a device 100 for treating urinary incontinence are shown, in accordance with an exemplary embodiment of the invention. In some embodiments of the invention, applicator 300 is any of those described in related applications PCT/IL2004/000433; PCT/IL2005/000304; PCT/IL2005/000303; PCT/IL2006/000346; PCT/IL2007/ 000893; PCT/IL2008/001292; U.S. Provisional Patent Application No. 60/719,422; U.S. Provisional Patent Application No. 60/762,059; and, U.S. Provisional Patent Application No. 60/960,492. Applicator 300 comprises a housing 302 and a plunger 304, in an exemplary embodiment of the invention. Housing 302 is adapted for receipt and/or storage of device 100, in an embodiment of the invention. In some exemplary embodiments, plunger 304 is used to expel device 100 from housing 302 during device 100 deployment into a vagina. FIG. 3B is a cross-section of FIG. 3A which shows the configuration of applicator 300 more clearly, including the interface between housing 302 and plunger 304. In some exemplary embodiments of the invention, housing 302 is provided with an outlet 308. Optionally, outlet 308 is comprised of a plurality of flexible flaps ("petals") which are pushed open when device 100 is expelled from applicator 300. Outlet 308 is shown more clearly in FIG. 3C.

In the applicator, the device is positioned so that the anchoring element 102 comes out of the applicator 300 into the vagina first, followed by the support element 104, which comes out at the end of the deployment process. In an embodiment of the invention, device 100 is deployed to render support to the urethra. Optionally, the device 100 is deployed to render support to the bladder neck.

In an embodiment of the invention, all device 100 configurations have arms 112, 114 of the same shape and length (with respect to each configuration), regardless of the variance in the angular opening between the arms. Having variously configured devices with same shape and length arms, allows the use of a single applicator design for every device 100 configuration, in accordance with an embodiment of the invention.

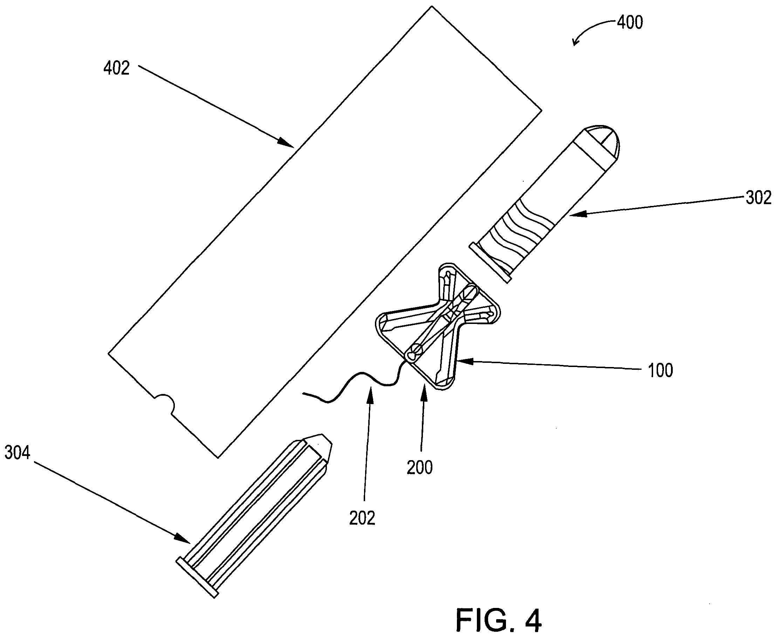

In an embodiment of the invention, the user receives the device in an individual package, shown and described in more detail with respect to FIG. 4. Optionally, different sizes and/or packages with devices having different features are color coded for ease of identification by consumers and/or user of the packages.

In an exemplary embodiment of the invention, insertion of the device into the vagina is similar to insertion of a tampon. The user uses one hand to spread the labia, directing the anterior part of the applicator 300 into the vagina, and compresses the plunger 304 with the other hand, thereby pushing the device from the applicator 300 into the vagina. In an exemplary embodiment of the invention, there is no need in a specific orientation because of the generally symmetrical design of device 100. The insertion can be performed at any orientation and/or anywhere in the 360° around the longitudinal axis 150 of device 100 in the applicator 300, in an embodiment of the invention. In some embodiments of the invention, even though the device is designed symmetrically, because each of the arms may flex independently of the other arms when *in situ* and each vagina varies in shape slightly, the device may not actually be in symmetrical form when in use (See FIG. 5). In certain cases, according to the user's decision, various lubricants may be used with the applicator for easier insertion.

FIG. 4 is a side view of the component parts of a product package 400 for a device 100 for treating urinary incontinence, in accordance with an exemplary embodiment of the invention. Product package 400 comprises applicator housing 302, device 100, cover 200, removal string 202, applicator plunger 304 and/or a product wrapper 402, in some embodiments of the invention. In an embodiment of the invention, product wrapper 402 is wrapped around the applicator 300, which when packaged includes device 100 within housing 302. Thus, product package 400 in its assembled form is more or less cylindrical or shaped like a wrapped tampon.

Referring to FIG. 5, a plan view showing a possible relationship of the support arms 114 to the central axis 150 of the device 100 when the device 100 is *in situ* is shown, in accordance with an exemplary embodiment of the invention. It is noted that not every vagina has the same contours and internal structure, even though some generalizations can be made about vaginal anatomical features. To that end, device 100 is designed to be adaptable to the varying vaginal features it may come across, depending on the user. For example, each of the arms 114 of the device 100 is capable of functioning independently (similar to independent shock absorber mechanisms for automobiles). This enables maximal flexibility, maximal adjustment to any vaginal shape (cross section) and/or vaginal dimensions. Thus, it may happen that in a particular cross section, the arms 114 of the device will not be symmetrically spread around the central axis 150 of the device 100, as shown in FIG. 5.

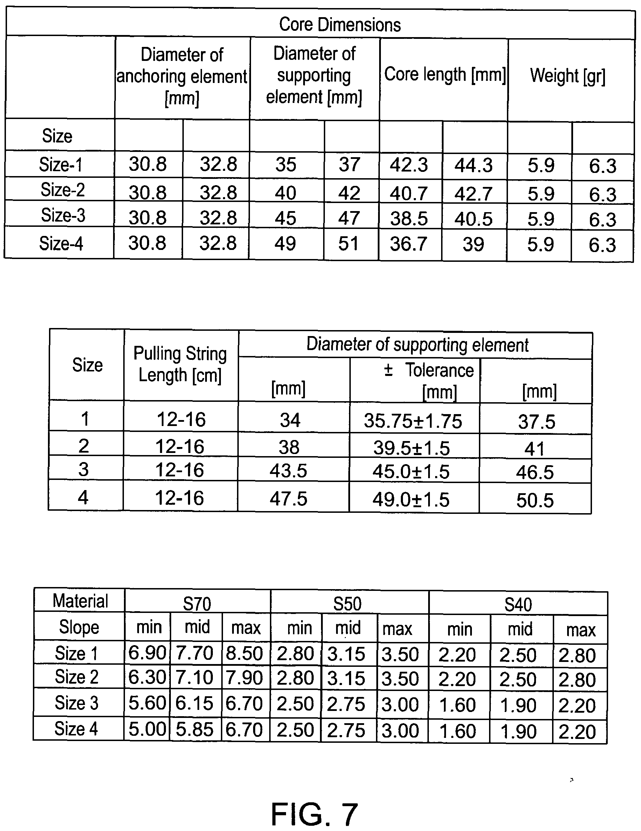

As described above, device 100 can be manufactured and/or commercially available in a plurality of configurations and/or sizes, with each configuration exhibiting different performance characteristics, operational dimensions, weight and the like. In some embodiments of the invention, differently configured devices 100 are made of the same material. Optionally, differently configured devices use different materials and/or different material ratios. FIG. 7 is a series of charts illustrating exemplary device configurations, in accordance with an exemplary embodiment of the invention. As seen in the chart, device 100 is produced in four configurations and/or sizes, in an embodiment of the invention, for optimal adjustment to various vaginal dimensions and/or in accordance with the severity of urinary incontinence. The various configurations differ in the diameter of the supporting arm's 114 spread and as a result, differ in the overall length when deployed (*i.e.* the arms 114 are spread). In an embodiment of the invention, the anchor arms 112 have identical deployed dimensions and/or performance characteristics in all of the configurations and/or sizes.

In some embodiments of the invention, device 100 is constructed of liquid silicone (LSR) by injection. It is possible to use other materials, for example TPE, non-liquid silicone and others for a device of the same configuration. In an embodiment of the invention, materials exhibiting various degrees of Shore hardness are used to produce softer or more rigid devices.

It should be understood that the various configuration combinations can be made varying the size and/or Shore hardness. For example, configurations 1 and 2, could be made with a Shore hardness of 70 while configurations 3 and 4 have a Shore hardness of 40. Optionally, all of the device configurations exhibit the same Shore hardness. Optionally, each individual configuration is made in multiple versions, each exhibiting different Shore hardness. In sum, various combinations and permutations of features, sizes, performance characteristics and/or construction materials can be employed to apply desired sub-urethral supporting force at certain working angles, in an embodiment of the invention.

In some embodiments of the invention the radiating support arms 114 of device 100 create an overall device diameter of 35mm to 51mm at the widest support element 114 cross section within the vaginal cavity. Optionally, the diameter is larger or smaller depending on the individual needs of the patient.

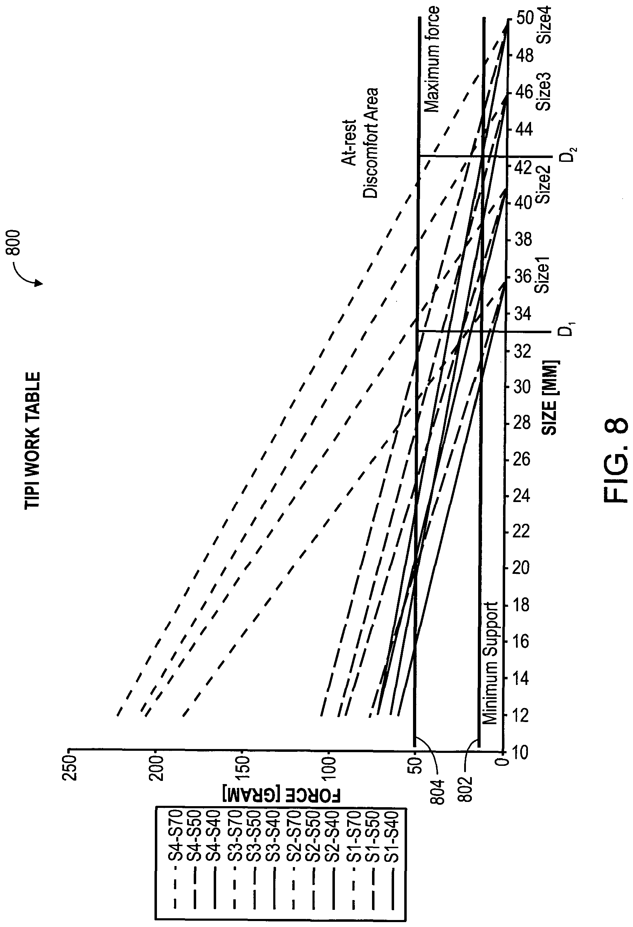

FIG. 8 is a device performance graph 800 correlating expansive force exerted by support arms 114 (y-axis) to amount of medial deflection (x-axis) and hardness (line hatchings) for each of the four basic configurations shown in FIG. 7, in accordance with an exemplary embodiment of the invention, and whereby medial deflection is the distance in mm towards the central longitudinal axis of the device from the natural expanded state of the arm.

The expansive force exerted by the supporting arms 114 is generally determined by the hardness grade and/or the medial flexion degree (medial deflection) of each of the supporting arms 114 relative to the central axis 150, in an embodiment of the invention. As described in other related applications (*e.g*. FIG. 5 of PCT/IL2006/000346), in some embodiments of the invention, an internal structure is included to assist with determining the medial deflection of each of the arms. If a specific material is used in construction, for example liquid silicone, these forces can be measured for any given diameter of the arms 114 of device 100, knowing the performance characteristics of the specific material being used. Using this data in a graph where the x-axis represents the device diameter and the y-axis represents the force, the forces exerted by the arms for a given device size and its material hardness grade is demonstrated, in an exemplary embodiment of the invention. The slope represents the ratio between the force (grams) and the amount of medial deflection (mm), in an embodiment of the invention.

In designing and/or selecting device 100 for use, certain performance considerations may be taken into account. It should be noted that device 100 support is "activated" by the supporting arms 114 being compressed (*i.e*. deflected towards the central axis 150 of the device 100, or "medial deflection") at least slightly by the vaginal wall. In general, the stronger the compressive forces on the supporting arms 114, the stronger the support force that is exerted back onto the vaginal wall/sub-urethra by the supporting arms 114. For example, supporting arms 114 must be compressed a certain minimal amount in order to provide counterforce sufficient for the supporting arms 114 to render support. That is, if a device 100 is inserted into a vagina and the device is too small or the angle of radial expansion is too small, then not enough force will be applied to the support arms 114 from the vaginal wall to cause the support arms 114 to counter with the force required to render appropriate and effective support. Failure to achieve this minimal value of compression in an at-rest state, shown as a horizontal minimal applied force line 802 in FIG. 8, on the supporting arms 114 during a stressful event will reduce device efficacy, in an embodiment of the invention. It should be noted that in some embodiments of the invention, the minimal applied force line 802 at 10 g is approximate and/or is variable ±3 grams.

Similarly, there exists is a maximum force exerted by the device on the vaginal walls beyond which the user would experience discomfort while the device is in the vagina at-rest and/or while removing the device, therefore in an embodiment of the invention, device 100 is designed and/or selected for use not to exceed this maximal force. This maximal force is represented in FIG. 8 as the bold horizontal line 804 at approximately 50 g of force. In an embodiment of the invention 50 grams is approximate and can vary ±5 grams. For example, if the radial expansion of the supporting arms 114 is too great, it will generate excessive force on the vaginal wall, the user may experience discomfort, which is to be avoided in an embodiment of the invention.

Graph 800 is used in an embodiment of the invention, to determine the use potential for a specific device configuration for a specific vaginal size. For example, D1 on graph 800 represents a vagina with a diameter of 33 mm. Referring to graph 800, it can be seen that Size 1 devices at S40 and S50 are not indicated for use with this patient because they will not provide sufficiently effective support in an at-rest state. However, a number of other device configurations are considered acceptable:
1. Use size 1 of shore 70 device (supplying force of ∼21 g)
2. Use size 2 of shore 50 device (supplying force of ∼25 g)
3. Use size 2 of shore 40 device (supplying force of ∼20 g)
4. Use size 3 of shore 50 device (supplying force of ∼38 g)
5. Use size 3 of shore 40 device (supplying force of ∼28 g)
6. Use size 4 of shore 50 device (supplying force of ∼48 g)
7. Use size 4 of shore 40 device (supplying force of ∼35 g)

As another example, D2 on graph 800 represents a vagina with a diameter of 42.5 mm. Because of the size of this patient's vagina, fewer choices are available to her for receiving ideally efficacious vaginal support. In this example, likely choices would include:
1. Use size 3 of shore 70 device (supplying force of ∼22 g)
2. Use size 4 of shore 70 device (supplying force of ∼45g)
3. Use size 4 of shore 50 device (supplying force of ∼22 g)
4. Use size 4 of shore 40 device (supplying force of ∼18 g)

It is noted that a size 4 device made of a low Shore hardness material, for example 40, can be used for a wide variety of vaginal diameters (approx. 30 mm to 45 mm), in an exemplary embodiment of the invention.

Above the maximal force line 804, graph 800 shows force exertion levels of the various device configurations at different levels of medial deflection all the way down to about 12 mm in total device diameter. In an embodiment of the invention, each arm is approximately 6 mm in width, therefore the minimum diameter distance possible is when two opposing arms have come into contact or 12 mm (6 mm + 6 mm). Specific numbers for selected device configurations are shown in and described in more detail with respect to FIG. 11, below.

FIG. 11 is a table showing specific performance levels for exemplary basic device configurations depicted in FIG. 8, in accordance with an exemplary embodiment of the invention. The minimal diameter column lists the diameter (in mm) at which two opposing arms come into contact thereby substantially prohibiting any further medial deflection by the arms. In an embodiment of the invention, maximal force is exerted when the devices are compressed the most, or at the minimal diameter. The max force column lists the maximum amount of force exerted by each listed configuration at the 12 mm diameter level, in an embodiment of the invention.

It is noted that some of the information shown in FIG. 11 is also included in the tables of FIG. 7. Where the information is not entirely in conformance, it should be understood that the broadest range of values is to be attributed to the inventions described herein. For example, a range of values may be taken where the low end of the range is from one table and the high end of the range is from another table. It should also be noted that values given are by way of example only, and that device performance characteristics can vary depending on materials used for construction and/or device configuration, *inter alia.*

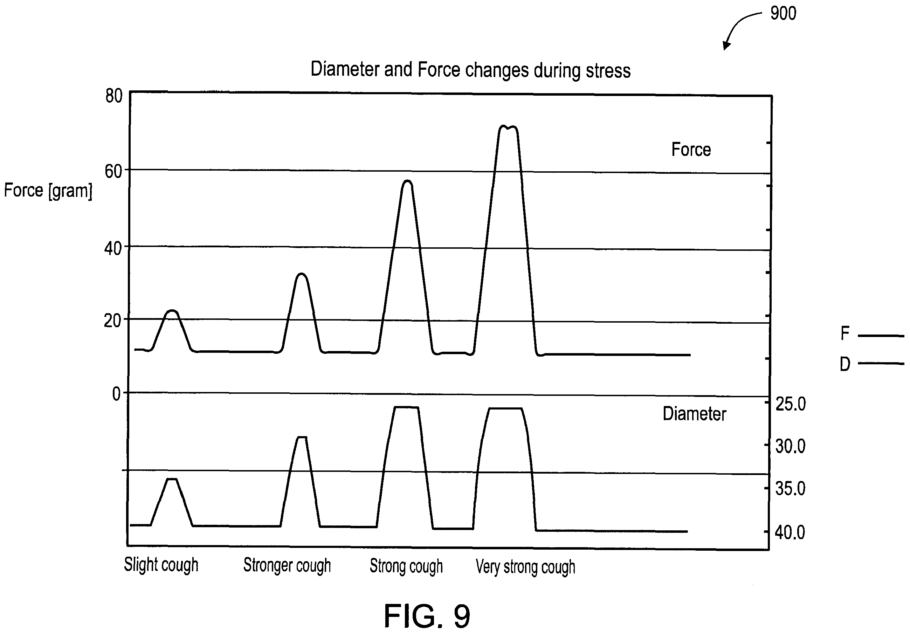

Referring to FIG. 9, a graph 900 correlating device diameter to force exerted during selected high stress moments is shown, in accordance with an exemplary embodiment of the invention. For the purposes of the study, a size 3 device of Shore 40 hardness with a slope of 1.9 g/mm and a nominal diameter of 46 mm was inserted into a vagina with an average diameter of 40mm. There are five (5) states which can be readily identified in graph 900:
1. In the normal at-rest state, the device opens and stabilizes itself within the vagina following insertion, under slightly compressive force so that its nominal diameter of 46 mm is slightly reduced by 0-3 mm per each opposite arm, in an embodiment of the invention. With a maximum medial deflection of approximately 6 mm, the force applied on the device by the vaginal wall and by the device on the vaginal wall in return is approximately 1.9 g/mm*6 mm=11.4g.
2. In a case of a slight cough, the vagina exerts approximately 10 grams of force on the device resulting in a decrease in device diameter by approximately 5.3 mm (10 g/1.9 g/mm) from the at-rest state. As shown in graph 900, once the high stress event is concluded, the device will return to its at-rest state with a diameter of 40 mm, in an embodiment of the invention.
3. In the case of a stronger cough, the vagina exerts approximately 20 grams of force on the device resulting in a decrease in device diameter by approximately 10.6 mm (20 g/1.9 g/mm) from the at-rest state.
4. In the case of a strong cough with a force of 45 g, the diameter of the device will decrease by a maximum of 20 mm (the initial 6 mm + an extra 14mm) from the at-rest state, in an embodiment of the invention. In an embodiment of the invention, the 14 mm extra is the maximum possible decrease as dictated by the configuration of the device and/or the point at which the support arms will come into contact with one another prohibiting any further reduction in diameter. The total force on the device is 56 g in this case, which is composed of the initial 11.4g + 45 g from the cough. By the end of this event, the device will return to its pre-cough diameter of 40 mm, in an embodiment of the invention.
5. In the case of a very strong cough, with a force of 60 g, the same maximum deflection of 20 mm will occur, from the at-rest state, and the total force on the device will be 71 g (initial 11.4 g+ 60 g from the cough). By the end of this event, the device will return to its pre-cough diameter of 40 mm, in an embodiment of the invention.

In certain situations, many of the possible sizes will be suitable for a particular patient and sufficient support can be provided without surpassing the discomfort threshold. This is particularly true with the soft devices of low Shore hardness and moderate slope, as described above. For example, a device of size 3 (support diameter-45 mm) made of soft silicone of 40 Shore may be suitable for a broad range of vaginal diameters: exerting a force of 12 grams over a diameter of 40 mm, 21 grams over a diameter of 35 mm, and 31 grams over a diameter of 30 mm. In other words, in some embodiments of the invention, a single device can provide support to numerous vaginal dimensions. In an embodiment of the invention, device 100 is adapted to be usable by a variety of users, for example by designing device 100 with a low or moderate slope.

It is possible that only one size will be suitable for a woman with a certain vaginal diameter, especially if her vaginal dimensions are larger than the average. In some embodiments of the invention, only sizes 3 or 4 would be suitable, while the smaller sizes would not exert the minimal support force required, in an embodiment of the invention. It should be noted that characteristics such as the force applied, the size and/or overall comfort of the device chosen is highly dependent on each individual user and may also depend on the pathological response caused by the device for each individual user.

In an exemplary embodiment of the invention, reducing the distance between the distal tip 118 of a supporting arm 114 and the central axis 150 of the device would increase the force exerted by the device 100 at the arm tip 118. The operative significance of this is that a relatively large device inserted into the vagina would exert a higher force on the vaginal walls as compared to a smaller device inserted into the same vagina. Using this rationale, if external forces exerted on the device reduce the internal distance between its arms 114 and the central axis 150 (*e.g*. during coughing, jumping, sneezing, etc.), a greater counteracting force would be exerted by the arms on the vaginal walls, thus enhancing the urethral support and the efficiency of urinary leak prevention. Therefore, in an exemplary embodiment of the invention, device 100 is designed with this activity-generated counter tension in mind.

### Experimental Results

### Patients

Sixty women with severe stress urinary incontinence ("SUI") were recruited into the study. Participants were women aged 18 to 70 years with severe SUI (scored 8 or more, based on Sandvik's four-level Severity Index) who would otherwise have been scheduled for a surgical procedure. They were familiar with the use of tampons, and had a normal Pap smear within the past 24 months.

Eligible women participated in an initial 7-day control period in which only pre-weighed pads were worn daily for 8 hours, followed by a 28-day device usage period in which both the device and pre-weighed pads were worn daily for 8 hours. Women were instructed to cough vigorously 10 times and to jump in place 50 times with a full bladder at some point during the day before voiding. Used pads were placed into a sealed bag, then collected and weighed by study staff within 30 h to determine the amount of pad weight gain ("PWG"). The first 14 days of the device usage period were used to familiarize women with how to use the device and to determine which of the 4 sizes of the device best suited them, beginning with the smallest. The last 14 days of the device usage period were used for comparison with the control period. The device was worn during the day for 8 h and disposed of after use. The study could be interrupted (i.e., nonconsecutive days of device usage were permitted) for women who were menstruating, had health problems not related to the device, or wished to use the device at their own pace and convenience.

Ten women (17%) eventually withdrew from the study: 4 because of a lack of interest, 4 due to adverse events, 1 because of an accompanying illness (the subject was treated with warfarin for cardiac arrhythmia and her physician advised her to withdraw despite having no bleeding problems), and 1 because of a de novo overactive bladder.

The mean age of the women was 50 years (range, 31 years to 70 years); most women were premenopausal (61.7%). Two postmenopausal women were receiving hormone replacement therapy at the start of the study. Twenty-five (41.7%) women had some degree of vaginal wall prolapse classified as ≤ grade 2 (above or at the level of the introitus). All women had severe SUI based on pre-study urodynamic testing.

### Efficacy outcomes

Fifty women (83%) used the device for the entire period and were included in the per-protocol population. Ninety-four percent of women achieved a ≥ 70% reduction in PWG from the control period to the last 14 days of device usage (*P* < 0.001). Mean PWG decreased from 16.85 g/ 8 h (range, 5.29 g/ 8 h to 40.55 g/ 8 h) during the control period to 1.96 g/ 8 h (range, 0.29 g/ 8 h to 4.79 g/ 8 h) during the last 14 days of device usage, with a statistically significant mean percent reduction of 86% (standard deviation, 9%; range, 59% to 98%; *P* < 0.0001). Over the entire 28-day device usage period, daily mean PWG decreased steadily during the first 6 days of device usage and remained stable at approximately 2 g/ 8 h from day 7 through the end of the device usage period. Reductions in PWG were observed for each individual participant during the device usage period.

Daily subjective perception of incontinence improved with device usage. During the control period, all women reported feeling incontinent, whereas only 8% felt incontinent by the end of the device usage period. Mean total scores on the Leak Score questionnaire assessing subjective perception of incontinence during effort-demanding activities decreased from 89.44 pre-study to 22.30 post-study (on a 0-100 scale) Device usage also improved quality of life based on the mean total scores from the IIQ-7 (41.89 pre-study to 4.41 post-study on a 0-100 scale) and the UDI-6 (48.22 pre-study to 11.56 post-study on a 0-100 scale). Women recorded a high degree of satisfaction with the device based on mean total scores from the satisfaction score questionnaire (29.18 out of a maximum score of 33 during the device usage period and 29.72 post-study). Possible responses to the satisfaction score questionnaire were "greatly", "moderate", "slightly", or "not at all", respectively scored as 3, 2, 1, and 0.

### Safety of the device

Urinary flow rates and post-void residual volumes were not significantly changed with device usage. Mean peak flow was 26.82 mL/s and 30.35 mL/s pre-study and during device usage, respectively (*P* = 0.141). Post-void residual volumes were 14.56 mL and 17.04 mL pre-study and during device usage, respectively (*P* = 0.536).

Symptomatic candidiasis was reported in 2 women during the study. In one case, candidiasis developed while taking antibiotics for follicular tonsillitis. Both women discontinued using the device and received appropriate treatment. The candidiasis was classified as unrelated to the device and resolved in both women by the end of the study. Asymptomatic candidiasis was reported in 4 other women at the post-study visit based on vaginal swabs. There were 4 cases of asymptomatic bacteriuria at the post-study visit: *Escherichia coli* (n = 1), *Proteus mirabilis* (n = 2), and *Klebsiella pneumoniae* (n = 1). There were no symptomatic urinary tract infections. Vaginal examinations at the end of the study showed no vaginal excoriations, sores, ulcers, scratches, or signs of inflammation.

The most common adverse events were genital tract discomfort, pain (considered to be a more intense feeling of discomfort), and spotting. No serious adverse events were reported. Of 102 total adverse events, 101 (99%) were mild and 96 (94%) were classified as probably related to the device. One adverse event was moderate and classified as not related to the device (one of the cases of candidiasis). Fourteen women (23%) reported spotting (a total of 24 adverse events), which appeared as minor, irregular blood stains on the cover of the device after removal. Spotting did not appear outside the vagina or on the pad in any of these cases. Four women withdrew due to adverse events (1 woman had 2 instances of spotting while using the device and 3 women had discomfort-1 of these women also had 2 instances of pain-while using the device). The number and percentage of patients with adverse events decreased over time with device usage. Adverse events occurred in only 97 (6.6%) out of 1475 participant-days (calculated as 28 days of device usage x 50 participants + 75 days of the ten women who withdrew from the study) during the device usage period.

### Overall assessment of Quality of Life

Stress urinary incontinence in women is an important health concern that affects daily life in various ways. The impact of SUI may be both physical and psychological with significant variability among women with regard to how incontinence is perceived. In some women, a single drop of leaked urine may cause considerable distress, whereas in others, larger amounts of leakage may go unnoticed. The perception of incontinence is a major factor in an individual's Quality of Life ("QoL").

Improvement in QoL was defined as any decrease in total or subscale scores from the pre-study visit to the mid - and post - study visits based on the IIQ-7, UDI-6, and Leak Score questionnaires. Between 88% and 100% of women exhibited decreases in scores from the pre-study visit to the mid- and post-study visits.

IIQ-7, the more accurate and suitable questionnaire for this kind of non-permanent device, showed a marked improvement between pre-study and post-study periods. 92% of the participants who used the device reported a QoL improvement of 10%, 88% reported a QoL improvement of 50%, and 70% of the women reported 90% improvement.

UDI-6 questionnaire is probably less favorable for this kind of study, as there was no significant change for the obstruction/discomfort subscale between pre-study and post-study periods, as dictated by the study protocol. 100% of the women had a 10% improvement, 86% had 50% improvement, but only 38% had a 90% improvement

The Leak Score, a validated investigator compiled questionnaire, reporting the subjective perception of wetness during various activities demonstrated a parallel trend, closer to the results of the IIQ-7 questionnaire, with 98% of the women reporting a 10% improvement, 92% reporting a 50% improvement and 58% of women reporting 90% improvement.

Significant improvement in QoL was demonstrated by the IIQ-7 and UDI-6 questionnaires. Mean total and all subscale scores of the IIQ-7 decreased significantly after using the device (all *P* values < 0.001). Statistically significant decreases in the mean total score of the UDI-6, as well as the irritation and stress subscale scores were observed by the end of study (all *P* values < 0.001). However, symptoms of obstruction/discomfort did not change significantly. This is not surprising since none of the women complained of voiding difficulties during the study and only those with normal urinary flow (without residual urine) could be enrolled.

Overall, the device was associated with a significant improvement in all aspects of QoL. Most women (92%) reported feeling dry and most (≥ 92%) reported improvement in QoL by the end of the study.

The IIQ-7 and UDI-6 results in the present study were similar to those described by Vassallo et al. for patients who underwent the TVT procedure. However, TVT, as well as TOT, are invasive procedures with well known QoL-associated adverse events, such as dyspareunia, voiding difficulties, de-novo urgency and urge incontinence, groin/thigh pains, etc. No such complaints were reported after using the device in the present study. The most common complaints in the present study were discomfort, pain, and spotting, which occurred primarily within the first few days of the study and diminished after women acclimated to the device.

Although the device is intended to support the urethra and treat SUI, beneficial effects on bladder storage function were also observed. Women often mentioned that irritative urinary symptoms, such as frequency, urgency, and leakage related to feelings of urgency, were significantly reduced. There were no reports of de novo urgency in this study.

The terms "comprises", "comprising", "includes", "including", "having" and their conjugates mean "including but not limited to". This term encompasses the terms "consisting of" and "consisting essentially of".

The phrase "consisting essentially of" means that the composition or method may include additional ingredients and/or steps, but only if the additional ingredients and/or steps do not materially alter the basic and novel characteristics of the claimed composition or method.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Although the invention has been described in conjunction with specific embodiments thereof, it is evident that many alternatives, modifications and variations will be apparent to those skilled in the art. Accordingly, it is intended to embrace all such alternatives, modifications and variations that fall within the scope of the appended claims.

## Claims

1. An apparatus (100) for insertion into a human vagina for treating urinary incontinence, comprising:
an intermediate section (106);
an anchoring section (102) located on one side of the intermediate section (106) provided with a plurality of beveled anchoring arms (112) adapted to prevent movement of the apparatus (100) into the vagina; and,
a supporting section (104) located on the opposite side of the intermediate section (106) from the anchoring section (102) provided with a plurality of beveled supporting arms (114) adapted to prevent movement of the apparatus (100) out of the vagina and to provide sub-urethral support;
**characterised in** further comprising a channel (120) positioned along a central axis (150) of the apparatus (100) and adapted for insertion of a rod therethrough, wherein the channel (120) is accessible by a rod through a tunnel (122) created by the beveled support arms (114) of the apparatus (100).

2. An apparatus (100) according to claim 1, wherein at least one of the anchoring arms (112) and the supporting arms (114) are provided with tips (118) adapted to induce the vaginal wall to envelop the tips (118) to prevent rotational motion of the apparatus (100) in the vagina.

3. An apparatus (100) according to claim 1 or claim 2, wherein the intermediate section (106) is adapted to conform to the longitudinal geometry of the vagina by being flexible around the natural arch of the vagina.

4. An apparatus (100) according to any of claims 1-3, wherein the tunnel (122) is a square tunnel (122) adapted for insertion of the rod therethrough, and is formed by the bevels of the plurality of supporting arms (114) when the supporting arms (114) are maximally compressed towards the central axis (150) of the apparatus (100).

5. An apparatus (100) according to any of claims 1-4, further comprising a cover (200), wherein the cover (200) encloses the intermediate, anchoring and supporting sections (106,102,104).

6. An apparatus (100) according to any of claims 1-5, wherein each of the plurality of arms (114) of the supporting section (104) supplies counter-force to the vaginal wall in proportion to the force applied to the arm (114) by the vaginal wall.

7. An apparatus (100) according to any of claims 1-6, further comprising an applicator (300) adapted to store and deploy the apparatus (100) into the vagina.

8. An apparatus (100) according to claim 7, wherein the beveling of the plurality of anchoring and supporting arms (112,114) is adapted to match the circumference of an applicator housing (302) of the applicator (300).

9. An apparatus (100) according to any of claims 1-8, wherein the plurality of beveled supporting arms (114) are arranged symmetrically around the central axis (150) of the device.

10. An apparatus (100) according to claim 9, wherein the apparatus (100) can be inserted into the vagina in any radial orientation around the central axis (150) to provide the sub-urethral support due to its symmetry.

11. An apparatus (100) according to any of claims 1-10, wherein each of the plurality of beveled supporting arms (114) is sufficiently flexible to twist at least one of clockwise and counter-clockwise around its own longitudinal axis.

12. An apparatus (100) according to any of claims 1-11, wherein the plurality of beveled supporting arms (114) are adapted to provide sub-urethral support by actively providing a counter-force to at least a vaginal wall in response to a high stress event which causes medial deflection to at least the supporting section of the apparatus (100).

13. An apparatus (100) for treating urinary incontinence according to any of claims 1-12, wherein the supporting section (104) renders between 10 grams and 50 grams of sub-urethral support at rest.

14. An apparatus (100) according to claim 13, wherein the supporting section (104) renders between 60 grams and 230 grams of sub-urethral support during a high stress event.

15. A method of packaging the apparatus (100) of any preceding claim, comprising:
inserting (602) a rod into the channel (120) in the apparatus (100) located along the central axis of the apparatus (100), wherein the channel (120) is accessed by the rod through the tunnel (122) created by the beveled supporting arms (114) of the apparatus (100);
urging (604) the apparatus towards an applicator housing (302) using the rod;
compressing (606) the supporting arms (114) of the apparatus (100) towards the central axis (150) while pushing (608) the apparatus into the applicator housing; and
retracting (610) the rod when the apparatus (100) is fully enclosed by the applicator housing (302).

## Patentansprüche

1. Vorrichtung (100) zum Einfügen in eine menschliche Scheide zum Behandeln von Harninkontinenz, aufweisend:
einen Zwischenabschnitt (106);
einen Verankerungsabschnitt (102), der sich auf einer Seite von dem Zwischenabschnitt (106) befindet, der mit einer Vielzahl von abgeschrägten Verankerungsarmen (112) versehen ist, die dazu angepasst sind, eine Bewegung von der Vorrichtung (100) in die Scheide zu verhindern; und,
einen Unterstützabschnitt (104), der sich auf der von dem Verankerungsabschnitt (102) gegenüberliegenden Seite von dem Zwischenabschnitt (106) befindet, der mit einer Vielzahl von abgeschrägten Unterstützarmen (114) versehen ist, die dazu angepasst sind, eine Bewegung von der Vorrichtung (100) aus der Scheide zu verhindern und eine suburethrale Unterstützung bereitzustellen;
**dadurch gekennzeichnet, dass** sie ferner einen Kanal (120) aufweist, der entlang einer zentralen Achse (150) von der Vorrichtung (100) positioniert ist und für ein dort hindurch Einfügen von einem Stab angepasst ist, wobei der Kanal (120) durch einen Stab durch einen durch die abgeschrägten Unterstützarme (114) von der Vorrichtung (100) geschaffenen Tunnel (122) zugänglich ist.

2. Vorrichtung (100) nach Anspruch 1, wobei wenigstens einer von den Verankerungsarmen (112) und den Unterstützarmen (114) mit Spitzen (118) versehen sind, die dazu angepasst sind, zu veranlassen, dass die Scheidenwand die Spitzen (118) umhüllt, um eine Drehbewegung von der Vorrichtung (100) in der Vagina zu verhindern.

3. Vorrichtung (100) nach Anspruch 1 oder Anspruch 2, wobei der Zwischenabschnitt (106) dazu angepasst ist, sich der longitudinalen Geometrie von der Scheide zu fügen, indem er um die natürliche Rundung der Scheide biegsam ist.

4. Vorrichtung (100) nach einem der Ansprüche 1-3, wobei der Tunnel (122) ein quadratischer Tunnel (122) ist, der für ein dort hindurch Einfügen von dem Stab angepasst, und durch die Schrägen von der Vielzahl von Unterstützarmen (114) gebildet ist, wenn die Unterstützarme (114) maximal in Richtung der zentralen Achse (150) von der Vorrichtung (100) zusammengedrückt sind.

5. Vorrichtung (100) nach einem der Ansprüche 1-4, weiter eine Hülle (200) aufweisend, wobei die Hülle (200) die Zwischen-, Verankerungs- und Unterstützabschnitte (106, 102, 104) umhüllt.

6. Vorrichtung (100) nach einem der Ansprüche 1-5, wobei jeder von der Vielzahl von Armen (114) von dem Unterstützabschnitt (104) eine Gegenkraft auf die Scheidenwand aufbringt, die proportional zu der durch die Scheidenwand auf den Arm (114) aufgebrachten Kraft ist.

7. Vorrichtung (100) nach einem der Ansprüche 1-6, weiter einen Applikator (300) aufweisend, der dazu angepasst ist, die Vorrichtung (100) aufzubewahren und in der Scheide zu installieren.

8. Vorrichtung (100) nach Anspruch 7, wobei die Abschrägung von der Vielzahl von Verankerungs- und Unterstützarmen (112, 114) dazu angepasst ist, dem Umfang von einem Applikatorgehäuse (302) von dem Applikator (300) zu entsprechen.

9. Vorrichtung (100) nach einem der Ansprüche 1-8, wobei die Vielzahl von abgeschrägten Unterstützarmen (114) symmetrisch um die zentrale Achse (150) von der Vorrichtung herum angeordnet sind.

10. Vorrichtung (100) nach Anspruch 9, wobei die Vorrichtung (100) in jeder radialen Ausrichtung um die zentrale Achse (150) herum in die Scheide eingefügt werden kann, um die suburethale Unterstützung bereitzustellen, aufgrund ihrer Symmetrie.

11. Vorrichtung (100) nach einem der Ansprüche 1-10, wobei jeder von der Vielzahl von abgeschrägten Unterstützarmen (114) ausreichend biegsam ist, um sich mindestens ein im Uhrzeigersinn und/oder Gegenuhrzeigersinn um seine eigene longitudinale Achse herum zu drehen.

12. Vorrichtung (100) nach einem der Ansprüche 1-11, wobei die Vielzahl von abgeschrägten Unterstützarmen (114) dazu angepasst sind, durch aktives Bereitstellen einer Gegenkraft auf wenigstens eine Scheidenwand als Reaktion auf ein hohes Belastungsereignis, das eine mediale Biegung von wenigstens dem Unterstützabschnitt von der Vorrichtung (100) bewirkt, eine suburethale Unterstützung bereitzustellen.

13. Vorrichtung (100) zum Behandeln von Harninkontinenz nach einem der Ansprüche 1-12, wobei der Unterstützabschnitt (104) in Ruhe zwischen 10 Gramm und 50 Gramm suburethale Unterstützung leistet.

14. Vorrichtung (100) nach Anspruch 13, wobei der Unterstützabschnitt (104) während einem hohen Belastungsereignis zwischen 60 Gramm und 230 Gramm suburethale Unterstützung leistet.

15. Verfahren zum Verpacken der Vorrichtung (100) nach einem vorhergehenden Anspruch, aufweisend:
Einfügen (602) eines Stabes in den Kanal (120) in der Vorrichtung (100), der sich entlang der zentralen Achse von der Vorrichtung (100) befindet, wobei der Kanal (120) durch den Stab durch den durch die abgeschrägten Unterstützarme (114) von der Vorrichtung (100) geschaffenen Tunnel (122) zugänglich ist;
Treiben (604) der Vorrichtung in Richtung eines Applikatorgehäuses (302) unter Verwendung des Stabes;
Zusammendrücken (606) der Unterstützarme (114) von der Vorrichtung (100) in Richtung der zentralen Achse (150) während einem Hineinschieben (608) der Vorrichtung in das Applikatorgehäuse; und
Zurückziehen (610) des Stabes, wenn die Vorrichtung (100) vollständig von dem Applikatorgehäuse (302) umschlossen ist.

## Revendications

1. Dispositif (100) pour l'insertion dans un vagin humain pour le traitement de l'incontinence urinaire, comprenant :
une section intermédiaire (106) ;
une section d'ancrage (102) située d'un côté de la section intermédiaire (106) munie d'une pluralité de bras d'ancrage biseautés (112) adaptés pour empêcher le déplacement du dispositif (100) dans le vagin ; et,
une section de support (104) située du côté opposé de la section intermédiaire (106) par rapport à la section d'ancrage (102) munie d'une pluralité de bras de support biseautés (114) adaptés pour empêcher le déplacement du dispositif (100) en dehors du vagin et pour fournir un support sous-urétral ;
**caractérisé en ce qu'**il comprend en outre un canal (120) positionné le long d'un axe central (150) du dispositif (100) et adapté pour l'insertion d'une tige à travers celui-ci, dans lequel le canal (120) est accessible par une tige à travers un tunnel (122) créé par les bras de support biseautés (114) du dispositif (100).

2. Dispositif (100) selon la revendication 1, dans lequel au moins un des bras d'ancrage (112) et des bras de support (114) sont munis d'extrémités (118) adaptées pour amener la paroi vaginale à envelopper les extrémités (118) pour empêcher le mouvement rotatif du dispositif (100) dans le vagin.

3. Dispositif (100) selon la revendication 1 ou la revendication 2, dans lequel la section intermédiaire (106) est adaptée pour se conformer à la géométrie longitudinale du vagin en étant flexible autour de la voûte naturelle du vagin.

4. Dispositif (100) selon l'une quelconque des revendications 1 à 3, dans lequel le tunnel (122) est un tunnel carré (122) adapté pour l'insertion de la tige à travers celui-ci, et est formé par les biseaux de la pluralité de bras de support (114) lorsque les bras de support (114) sont comprimés au maximum vers l'axe central (150) du dispositif (100).

5. Dispositif (100) selon l'une quelconque des revendications 1 à 4, comprenant en outre une enveloppe (200), dans lequel l'enveloppe (200) renferme les sections intermédiaire, d'ancrage et de support (106, 102, 104).

6. Dispositif (100) selon l'une quelconque des revendications 1 à 5, dans lequel chacun de la pluralité de bras (114) de la section de support (104) exerce une force contraire à la paroi vaginale proportionnellement à la force appliquée sur le bras (114) par la paroi vaginale.

7. Dispositif (100) selon l'une quelconque des revendications 1 à 6, comprenant en outre un applicateur (300) adapté pour conserver et déployer le dispositif (100) dans le vagin.

8. Dispositif (100) selon la revendication 7, dans lequel le biseautage de la pluralité de bras d'ancrage et de support (112, 114) est adapté pour correspondre à la circonférence d'un logement d'applicateur (302) de l'applicateur (300).

9. Dispositif (100) selon l'une quelconque des revendications 1 à 8, dans lequel la pluralité de bras de support biseautés (114) sont disposés symétriquement autour de l'axe central (150) du dispositif.

10. Dispositif (100) selon la revendication 9, dans lequel le dispositif (100) peut être inséré dans le vagin selon une quelconque orientation radiale autour de l'axe central (150) pour fournir le support sous-urétral du fait de sa symétrie.

11. Dispositif (100) selon l'une quelconque des revendications 1 à 10, dans lequel chacun de la pluralité de bras de support biseautés (114) est suffisamment flexible pour se tordre dans au moins l'un parmi le sens horaire et le sens antihoraire autour de son propre axe longitudinal.

12. Dispositif (100) selon l'une quelconque des revendications 1 à 11, dans lequel la pluralité de bras de support biseautés (114) sont adaptés pour fournir un support sous-urétral en fournissant une force contraire sur au moins une paroi vaginale en réponse à un événement à contraintes élevées qui entraîne une déflexion médiale au moins à la section de support du dispositif (100).

13. Dispositif (100) de traitement de l'incontinence urinaire selon l'une quelconque des revendications 1 à 12, dans lequel la section de support (104) apporte entre 10 grammes et 50 grammes de support sous-urétral au repos.

14. Dispositif (100) selon la revendication 13, dans lequel la section de support (104) apporte entre 60 grammes et 230 grammes de support sous-urétral pendant un événement à contraintes élevées.

15. Procédé de conditionnement du dispositif (100) selon une quelconque revendication précédente, comprenant :
l'insertion (602) d'une tige dans le canal (120) dans le dispositif (100) situé le long de l'axe central du dispositif (100), dans lequel le canal (120) est accessible par la tige à travers le tunnel (122) créé par les bras de support biseautés (114) du dispositif (100) ;
la poussée (604) du dispositif vers un logement d'applicateur (302) à l'aide de la tige;
la compression (606) des bras de support (114) du dispositif (100) vers l'axe central (150) tout en poussant (608) le dispositif dans le logement d'applicateur ; et
la rétractation (610) de la tige lorsque le dispositif (100) est entièrement renfermé dans le logement d'applicateur (302).
